(19)

Europäisches
Patentamt
European
Patent Office
Office européen
des brevets

(11) **EP 2 181 642 A1**

(12) **EUROPEAN PATENT APPLICATION**
published in accordance with Art. 153(4) EPC

(43) Date of publication:
**05.05.2010 Bulletin 2010/18**

(51) Int Cl.:
**A61B 1/04** *(2006.01)*  **A61B 1/00** *(2006.01)*

(21) Application number: **08765114.7**

(22) Date of filing: **04.06.2008**

(86) International application number:
**PCT/JP2008/060301**

(87) International publication number:
**WO 2009/025115 (26.02.2009 Gazette 2009/09)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **23.08.2007 JP 2007217578**

(71) Applicant: **Olympus Corporation
Tokyo 151-0072 (JP)**

(72) Inventor: **MATSUDA, Takehiro
Tokyo 151-0072 (JP)**

(74) Representative: **Gunzelmann, Rainer et al
Wuesthoff & Wuesthoff
Patent- und Rechtsanwälte
Schweigerstraße 2
81541 München (DE)**

(54) **IMAGE PROCESSING APPARATUS, IMAGE PROCESSING PROGRAM, AND IMAGE PROCESSING METHOD**

(57) Provided are an image information storage unit (121) that stores an image, an image display unit (131) that sequentially displays each image, a feature-information storage unit (122) that stores feature information of each image, a feature-information display unit (132) that displays the feature information, a skip indicator receiving unit (141) that receives a skip indicator, which is an indicator for skipping a display of the image on the image display unit (131), a skip instruction receiving unit (142) that receives an instruction to skip the image displayed on the image display unit (131), and an image display control unit (112) that controls the image display unit (131) to skip the image displayed on the image display unit (131) according to the feature information and based on the skip indicator when the instruction to skip an image is received.

FIG.2

**Description**

TECHNICAL FIELD

The present invention relates to an image processing apparatus that displays an image, an image processing program which can be provided as a computer program product, and an image processing method.

BACKGROUND ART

In recent years, a swallowable capsule endoscope which is swallowed by a patient, i.e., a subject from the mouth, and introduced inside the subject is proposed as an imaging device which picks up an image inside a living body. The capsule endoscope picks up several tens of thousands of in-vivo images in, for example, an esophagus, a stomach, a small intestine, and a large intestine, after being swallowed from the mouth of the patient until naturally excreted. A doctor, a nurse, or others (referred to below as "examiner"), who observe the images and diagnose the patient make the picked-up in-vivo images taken into an image processing apparatus having an image display function and observe the in-vivo images.

Conventionally, there has been known an image processing apparatus which sequentially displays in-vivo images in an order of time series according to an instruction of an operator or the like (see Patent Document 1). The examiner observes the in-vivo images sequentially displayed by the image processing apparatus. When an image which is necessary for the diagnosis of the subject is displayed, the examiner gives an instruction to the operator. The operator watches play time of the image for which the examiner gives the instruction, and makes the image corresponding to the play time displayed as a still image. Then, the examiner watches carefully and observes the image displayed as a still image.

Patent Document 1: Japanese Patent Application Laid-Open 2006-330376
Patent Document 2: Japanese Patent Application Laid-Open 2006-280792
Patent Document 3: Japanese Patent Application Laid-Open No. 2006-288879
Nonpatent Literature 1: Digital Image Processing, CG-ARTS Society, July 22, 2004, P.202
Nonpatent Literature 2: Digital Image Processing, CG-ARTS Society, July 22, 2004, P.181

DISCLOSURE OF INVENTION

PROBLEM TO BE SOLVED BY THE INVENTION

With the conventional image processing apparatus, after an observer such as an examiner visually recognizes an image worth careful viewing, i.e., an image corresponding to desired feature information, and gives an instruction to an operator, a time passes until the operator confirms a play time and stops the image. Hence, manipulations such as image rewinding are required for replaying the image corresponding to the desired feature information. Further, though the conventional image processing apparatus is efficient when the operator is present, it is difficult for the examiner manipulating the conventional image processing apparatus alone to visually recognize the image corresponding to the desired feature information and confirming the play time corresponding to the image at the same time.

Further, with the conventional image processing apparatus, an observer such as an examiner determines whether each image has desired feature information or not by actually visually recognizing the image such as sequentially-displayed in-vivo images. Hence, no image processing apparatus has been proposed which supports the determination as to whether each image is an image corresponding to desired feature information or not, and displays the image corresponding to the desired feature information in an efficient manner.

The present invention has been made in view of the above and an object of the present invention is to provide an image processing apparatus, image processing program, and image processing method that can display an image corresponding to desired feature information according to an instruction by an observer in an efficient manner while skipping display of images other than the image corresponding to the desired feature information, among a series of images.

MEANS FOR SOLVING PROBLEM

To solve the problems as described above and to achieve an object, an image processing apparatus according to the present invention includes an image storage unit that stores an image, an image display unit that sequentially displays each image, a feature-information storage unit that stores feature information of each image, a feature-information display unit that displays the feature information, a skip indicator receiving unit that receives a skip indicator, which is an indicator set in relation to the feature information to skip a display of the image on the image display unit, a skip instruction receiving unit that receives an instruction to skip the image displayed on the image display unit, and an image display control unit that controls the image display unit to skip the image displayed on the image display unit according to the feature information and based on the skip indicator when the skip instruction receiving unit receives the instruction to skip an image.

Further, an image processing program according to the present invention is an image processing program for reading each of images stored in an image storage unit and sequentially displaying the images on an image display unit, and causes a computer to perform a feature information storing step of storing feature information of each of the images stored in the image storage unit, in a feature-information storage unit, a feature information displaying step of displaying the feature information, a skip indicator receiving step of receiving a skip indicator, which is an indicator set in relation to the feature information to skip a display of the image on the image display unit, a skip instruction receiving step of receiving an instruction to skip the image displayed on the image display unit, and an image display controlling step of controlling the image display unit to skip the image displayed on the image display unit according to the feature information and based on the skip indicator when the instruction to skip the image is received in the skip instruction receiving step.

Further, an image processing method according to the present invention is an image processing method for reading each of images stored in an image storage unit and sequentially displaying the images on an image display unit, and includes a feature information storing step of storing feature information of each of the images stored in the image storage unit, in a feature-information storage unit, a feature information displaying step of displaying the feature information, a skip indicator receiving step of receiving a skip indicator, which is an indicator set in relation to the feature information to skip a display of the image on the image display unit, a skip instruction receiving step of receiving an instruction to skip the image displayed on the image display unit, and an image display controlling step of controlling the image display unit to skip the image displayed on the image display unit according to the feature information and based on the skip indicator when the instruction to skip the image is received in the skip instruction receiving step.

EFFECT OF THE INVENTION

The image processing apparatus according to the present invention skips image display based on the feature information of the image when receiving a skip instruction from an observer, according to the skip indicator set in relation to the feature information of the image. Therefore, when the observer determines that a portion of the series of images is not worth observation based on the image and the feature information, the observer can skip the display of the images other than those images corresponding to the desired feature information and observe the images corresponding to the desired information for sure.

BRIEF DESCRIPTION OF DRAWINGS

FIG. 1 is a diagram of an overall configuration of an intra-subject information acquiring system according to a first embodiment of the present invention.
FIG. 2 is a block diagram of a configuration of an image processing apparatus according to the first embodiment of the present invention.
FIG. 3 is a view of an example of a display screen of a display unit shown in FIG. 2.
FIG. 4 is an enlarged view of a feature-information-graph display area shown in FIG. 3.
FIG. 5 is a flowchart of a procedure of a display process to display a series of in-vivo images, performed by the image processing apparatus shown in FIG. 2.
FIG. 6 is a block diagram of a configuration of an image processing apparatus according to a second embodiment of the present invention.
FIG. 7 is a view of an example of a display screen of a display unit shown in FIG. 6.
FIG. 8 is an enlarged view of a feature-information-graph display area shown in FIG. 7.
FIG. 9 is a flowchart of a procedure of a display process to display a series of in-vivo images, performed by the image processing apparatus shown in FIG. 6.

EXPLANATIONS OF LETTERS OR NUMERALS

| 1 | Subject |
|---|---|
| 2 | Capsule endoscope |
| 3 | Receiving apparatus |
| 4 | Recording medium |
| 5, 6 | Image processing apparatus |
| 10, 20 | Control unit |
| 11 | Card interface (I/F) |
| 12, 22 | Storage unit |
| 13, 23 | Display unit |
| 14, 24 | Input unit |
| 101, 201 | Feature-information calculating unit |
| 102 | Image-similarity calculating unit |
| 103 | Nontarget-tissue existence-ratio calculating unit |
| 111, 211 | Display control unit |
| 112, 212 | Image display control unit |
| 113, 213 | Feature-information display control unit |
| 121 | Image information storage unit |
| 122, 222 | Feature-information storage unit |
| 123 | Image-similarity storage unit |
| 124 | Nontarget-tissue existence-ratio storage unit |
| 131 | Image display unit |
| 132, 232 | Feature-information display unit |
| 141, 241 | Skip indicator receiving unit |
| 142 | Skip instruction receiving unit |
| 203 | Lesion-existence-probability calculating unit |
| 224 | Lesion-existence-probability storage unit |
| W1, W2 | Diagnosis window |
| F1 | Image display area |
| F2 | Image ID information display area |
| F3 | Forward button |
| F4 | Backward button |
| F5 | Skip forward button |
| F6 | Skip backward button |
| F7, F15 | Feature-information-graph display area |
| F8 | Image-similarity transition curve |
| F9 | Nontarget-tissue existence-ratio transition curve |
| F10 | Image-similarity skip indicator |
| F11 | Play position |
| F12 | Skip forward position |
| F13 | Skip backward position |
| F14 | Image-similarity skip indicator (before change) |
| F16 | Lesion-existence-probability transition curve |
| F17 | Lesion-existence-probability skip indicator |

BEST MODE(S) FOR CARRYING OUT THE INVENTION

Exemplary embodiments of an image processing apparatus, an image processing program, and an image processing method according to the present invention are described below with reference to the accompanying drawings. The present invention is not limited to the embodiments.

First Embodiment

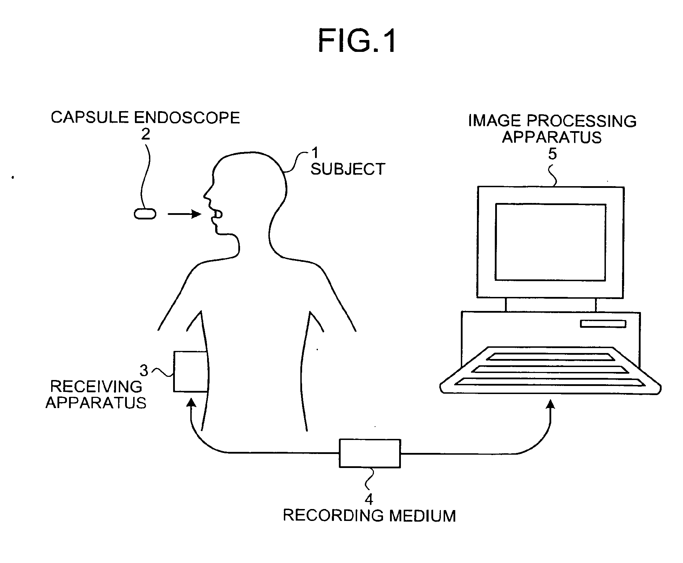

FIG. 1 is a schematic diagram of a configuration of an intra-subject information acquiring system including an image processing apparatus according to a first embodiment of the present invention. As shown in FIG. 1, the intra-subject information acquiring system includes a capsule endoscope 2, a receiving apparatus 3, an image processing apparatus 5, and others. The capsule endoscope 2 picks up in-vivo images inside a subject 1. The receiving apparatus 3 receives image information of the in-vivo images radio transmitted from the capsule endoscope 2. The image processing apparatus 5 processes the in-vivo images picked up by the capsule endoscope 2 based on the image information received by the receiving apparatus 3. For transfer of the image information between the receiving apparatus 3 and the image processing apparatus 5, a recording medium 4 is employed.

The capsule endoscope 2, which is introduced inside the subject 1, has an imaging function to sequentially pick up images inside the subject 1 in time series, and a radio communication function to transmit radio signals including the picked-up images to an outside. The capsule endoscope 2 is swallowed by the subject 1, advances in the living body following peristaltic movements of a gastrointestinal tract, sequentially picks up images inside the subject 1 at predetermined intervals, for example, at 0.5-second intervals, and sequentially transmits the images inside the subject 1 to the receiving apparatus 3 through predetermined electric waves.

The receiving apparatus 3 sequentially stores in the recording medium 4 information such as a received image, and imaging time which indicates time elapsed since the capsule endoscope 2 is introduced into the subject 1 until each image is picked up, as image information. The recording medium 4 is realized with a portable recording medium such as a CompactFlash®. The recording medium 4 is attachable/detachable to/from the receiving apparatus 3 and the image processing apparatus 5, and has such a configuration that the information can be output therefrom and recorded therein when attached to the receiving apparatus 3 and the image processing apparatus 5.

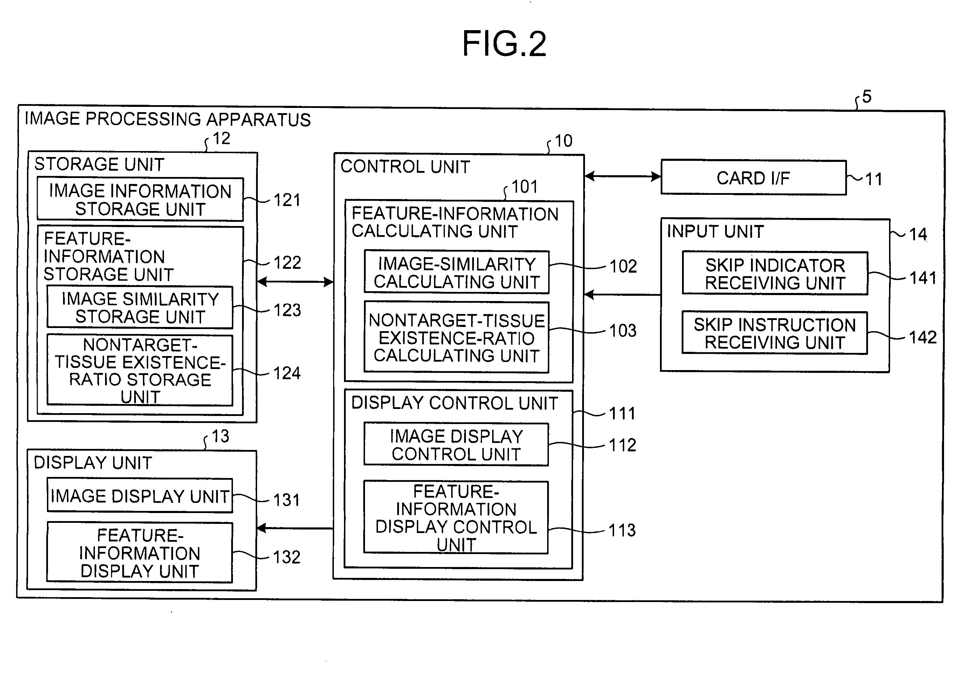

The image processing apparatus 5 has an image display function to take in the image information stored in the recording medium 4 by the receiving apparatus 3, and to sequentially display the in-vivo images of the subject 1 each for a predetermined display time (such a manner of display is referred to below as "play"). The examiner makes the image processing apparatus 5 play the in-vivo images, and observes (i.e. examines) an interior of the living body of the subject 1, such as an esophagus, a stomach, a small intestine, and a large intestine. The image processing apparatus 5 is configured in a similar manner to a workstation. Specifically, the image processing apparatus 5 includes a control unit 10, a card interface (I/F) 11, a storage unit 12, a display unit 13, and an input unit 14 as shown in FIG. 2.

The control unit 10 is realized with a CPU or the like. The control unit 10 controls various kinds of processes performed by each unit of the image processing apparatus 5, and controls input/output of information among the units of the image processing apparatus 5. The control unit 10 includes a feature-information calculating unit 101 that calculates feature information of each image by processing the image information, and a display control unit 111 that controls a display process in the display unit 13.

The feature-information calculating unit 101 includes an image-similarity calculating unit 102 and a nontarget-tissue existence-ratio calculating unit 103. The feature-information calculating unit 101 calculates an image similarity and a nontarget-tissue existence ratio of each image as the feature information. The image-similarity calculating unit 102 calculates the image similarity which indicates a degree of similarity between predetermined images in a series of in-vivo images, for example, between successive time-series images. The nontarget-tissue existence-ratio calculating unit 103 calculates the nontarget-tissue existence ratio which indicates a ratio of tissues such as a stool and a bubble in an image area, other than organ tissues which the examiner intends to observe, such as a mucous surface and a villus. The control unit 10 may acquire the feature information calculated by the examiner or by another image processing apparatus via the card I/F 11 or the input unit 14. In this case, the feature-information calculating unit 101 is not needed.

The display control unit 111 controls the display of various kinds of information, and determines an image whose display is to be skipped, that is, a skipped image, based on a skip indicator. Here, to skip the display of an image means that a predetermined image is not displayed or displayed at high speed while the series of in-vivo images is displayed in an order of time series or in a reverse order of time series, in short, it means that a predetermined image is skipped during the display. The display control unit 111 includes an image display control unit 112 and a feature-information display control unit 113. The image display control unit 112, on receiving an input of a play instruction, displays the series of in-vivo images on the display unit 13 by playing the series of in-vivo images. Further, on receiving an instruction to skip the display of an image, in other words, on receiving an input of a skip instruction, the image display control unit 112 controls the display to skip the display of an image which is determined to be the skipped image based on the skip indicator by the display control unit 111. The feature-information display control unit 113 controls the display to display the feature information on the display unit 13.

The card I/F 11, to which the recording medium 4 is removably attached, reads out image information and image ID information stored in the recording medium 4 and transfers the read-out information to the control unit 10. Further, the card I/F 11 writes into the recording medium 4 information for which the control unit 11 gives a write instruction, for example, the image ID information. The image ID information includes, for example, a name, a sex, and a birth date of the subject 1, and an image ID.

The storage unit 12 is realized with an information recording medium in which information can be stored and from which information can be read out, such as a random access memory (RAM), an electrically erasable programmable read-only memory (EEPROM), and a hard disk. The storage unit 12 stores information for which the control unit 10 gives a write instruction, and supplies information for which the control unit 10 gives a read instruction to the control unit 10. The storage unit 12 includes an image information storage unit 121 that stores the image ID information corresponding to the series of in-vivo images and the image information of the series of in-vivo images, and a feature-information storage unit 122 that stores the feature information of each image stored in the image information storage unit 121. The feature-information storage unit 122 includes an image-similarity storage unit 123 that stores the image similarity of each image, and a nontarget-tissue existence-ratio storage unit 124 that stores the nontarget-tissue existence ratio of each image.

The display unit 13 is realized with various kinds of displays such as a CRT display and a liquid crystal display, and displays various kinds of information for which the display control unit 111 gives a display instruction. In particular, the display unit 13 includes an image display unit 131 that displays the in-vivo image, and a feature-information display unit 132 that displays the feature information, and displays various kinds of information necessary for observation and diagnosis of the inside of the living body of the subject 1.

The input unit 14 is realized with a keyboard, a mouse, and the like, and inputs various kinds of information to the control unit 10 according to an input manipulation by the examiner. Further, the input unit 14 inputs various kinds of information via a GUI (Graphical User Interface) displayed on the display unit 13. The input unit 14 includes a skip indicator receiving unit 141 that receives an input of a skip indicator, which is an indicator to determine the skipped image, and a skip instruction receiving unit 142 that receives an input of a skip instruction for the image display control unit 112.

The CPU in the image processing apparatus which includes the units as described above reads out from the storage unit 12 an image processing program for realizing processes performed by the image processing apparatus of the embodiment and executes the same. The image processing program can be recorded in a computer-readable recording medium such as a flexible disc, a CD-ROM, a DVD-ROM, and a flash memory so as to be widely distributed. Therefore, the image processing apparatus according to the embodiment may include an auxiliary storage device which can read out information from at least one of various types of recording medium as listed above.

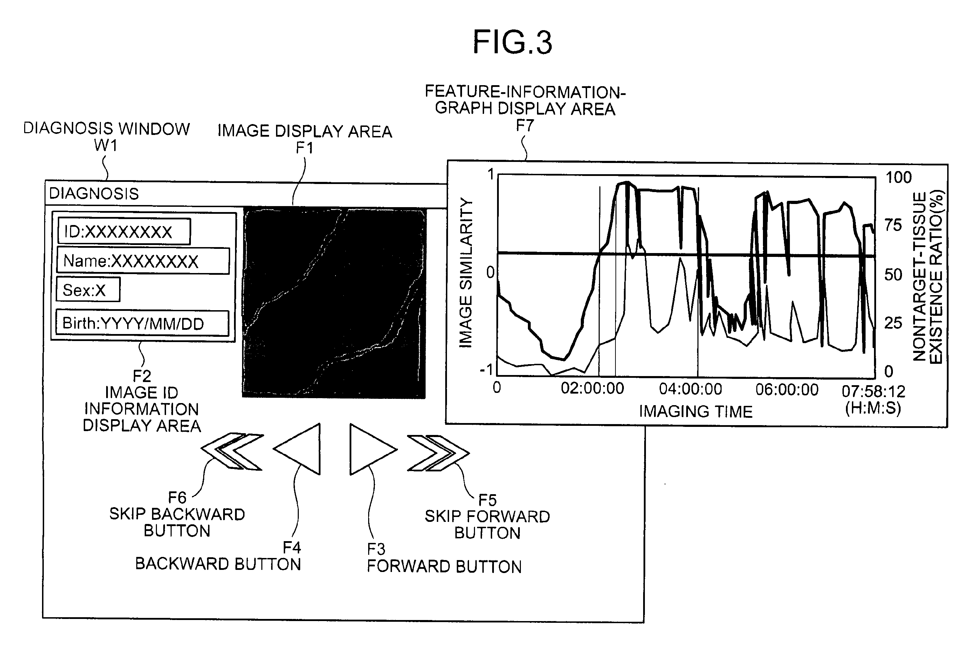

Next, a specific example of a display screen of the display unit 13 is described with reference to FIG. 3. FIG. 3 is a view of a diagnosis window W1, which is an example of the display screen. The diagnosis window W1 includes an image display area F1 in which the in-vivo image is displayed by playing, an image ID information display area F2 in which the image ID information is displayed, a forward button F3 and a backward button F4, a skip forward button F5 and a skip backward button F6, and a feature-information-graph display area F7. Here, the image display area F1 represents a specific mode of the image display unit 131, and the feature-information-graph display area F7 represents a specific mode of the feature-information display unit 132.

The diagnosis window W1 is a GUI screen, where the examiner clicks and selects the forward button F3 or the backward button F4 using a mouse (not shown) of the input unit 14 to input the play instruction for the series of in-vivo images to the display control unit 111. When the examiner selects the forward button F3, the image display control unit 112 displays each image for a predetermined display time in an order of imaging time-series, whereas when the examiner selects the backward button F4, the image display control unit 112 displays each image for a predetermined display time in a reverse order of imaging time-series. While the image is displayed (i.e., played), the selected button changes into a play stop button not shown. The examiner selects the play stop button to input a play-stop instruction to the display control unit 111.

Further, the examiner can skip the display of a predetermined image by selecting the skip forward button F5 or the skip backward button F6. The skip forward button F5, the skip backward button F6, and the mouse not shown represent a specific mode of the skip instruction receiving unit 142. When the skip forward button F5 is selected, the image display control unit 112 skips the display of images subsequent to an image displayed in the image display area F1 in the order of imaging time-series up to an image not to be skipped, based on the skip indicator. On the other hand, when the skip backward button F6 is selected, the image display control unit 112 skips the display of images previous to the image displayed in the image display area F1 in a reverse order of imaging time-series up to an image not to be skipped.

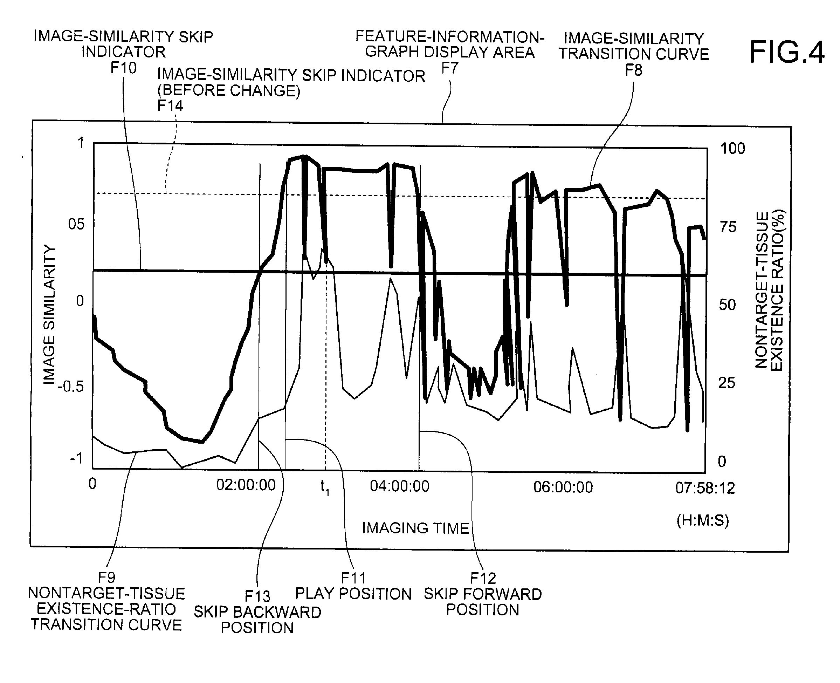

In the feature-information-graph display area F7, a graph showing a relation between information specifying each image in the time series of the entire series of in-vivo images and feature information of each image is displayed under the control of the feature-information display control unit 113. For example, in the feature-information-graph display area F7, an image-similarity transition curve F8 and a nontarget-tissue existence-ratio transition curve F9 are displayed as shown in FIG. 4, where the imaging time is plotted on a horizontal axis, and the image similarity and the nontarget-tissue existence ratio of each image are plotted on a vertical axis. The image-similarity transition curve F8 indicates a relation between the imaging time and the image similarity, and the nontarget-tissue existence-ratio transition curve F9 indicates a relation between the imaging time and the nontarget-tissue existence ratio.

The information specifying each image in the time series is not limited to the imaging time, and may be, for example, play time which indicates time elapsed since the playing starts until each image is displayed while the series of in-vivo images is played, or may be an image number attached to each image in an order of imaging.

In the feature-information-graph display area F7, an image-similarity skip indicator F10 is displayed as the skip indicator in a form of a line parallel to the horizontal axis. The display control unit 111 determines an image whose image similarity is higher than the image-similarity skip indicator F10 to be the skipped image.

When the image similarity of the time-series images is continuously high, images of a similar content are displayed in succession. In this case, the examiner can grasp the condition of the living body which appears in the images only by watching some of the images. Therefore, when a part of the images is displayed among the images with high image similarity, the display of the rest can be skipped.

The image similarity can change due to a change of an imaged position of the living body, or an emergence of a lesion tissue, and moreover possibly due to an emergence of a tissue which is not a target of observation, a subtle change of a position of the capsule endoscope 2, or a change of a form of an organ tissue. For example, at imaging time $t_1$, the image similarity sharply falls and the nontarget-tissue existence ratio rises at the same time. Therefore, the examiner can determine that the image similarity sharply falls because of an emergence of a nontarget tissue in the image and not because of the change in the imaged position in the living body or the like.

When the examiner determines that it is not necessary to observe an image corresponding to the imaging time $t_1$, the examiner can skip the display of the image by setting the image-similarity skip indicator F10 lower than the image similarity at the imaging time $t_1$.

As a specific manipulation, the examiner drags an image-similarity skip indicator F14 downward along the vertical axis using the mouse not shown. The examiner drops the dragged image-similarity skip indicator F14 at a position of the image-similarity skip indicator F10, in order to set the image-similarity skip indicator lower than the image similarity of the image corresponding to the imaging time $t_1$. The image-similarity skip indicator F10 and the mouse not shown represent a specific mode of the skip indicator receiving unit 141. As described above, the examiner compares the feature information of an image currently displayed in the image display area F1 with the feature information of each image, sets the image-similarity skip indicator indicating a threshold value of the image similarity to determine the skipped image, and determines the skipped image.

A play position F11 in the feature-information-graph display area F7 is a line parallel to the vertical axis, and is an indicator which moves along the horizontal axis, that is, an imaging time axis, to indicate imaging time corresponding to an image currently displayed in the image display area F1. The feature-information display control unit 113 controls and moves the play position F11 according to the image currently displayed in the image display area F1.

A skip forward position F12 is an indicator indicating imaging time corresponding to an image that is to be displayed after the currently displayed image in the display area F1 when the skip forward button F5 is currently selected. In other words, the skip forward position F12 represents imaging time corresponding to a skip destination image. As shown in FIG. 4, an image corresponding to the skip forward position F12 is an image at which the image similarity is lower than the image-similarity skip indicator F10 for the first time after the play position F11 in a forward direction of the imaging time-series. On the other hand, a skip backward position F13 is an indicator indicating imaging time corresponding to a skip destination image when the skip backward button F6 is currently selected. As shown in FIG. 4, an image corresponding to the skip backward position F13 is an image at which the image similarity is lower than the image-similarity skip indicator F10 for the first time before the play position F11 in a backward direction of the imaging time-series. The skip forward position F12 and the skip backward position F13 are lines parallel to the vertical axis, similarly to the play position F11, and move along the horizontal axis representing the imaging time under the control of the feature-information display control unit 113.

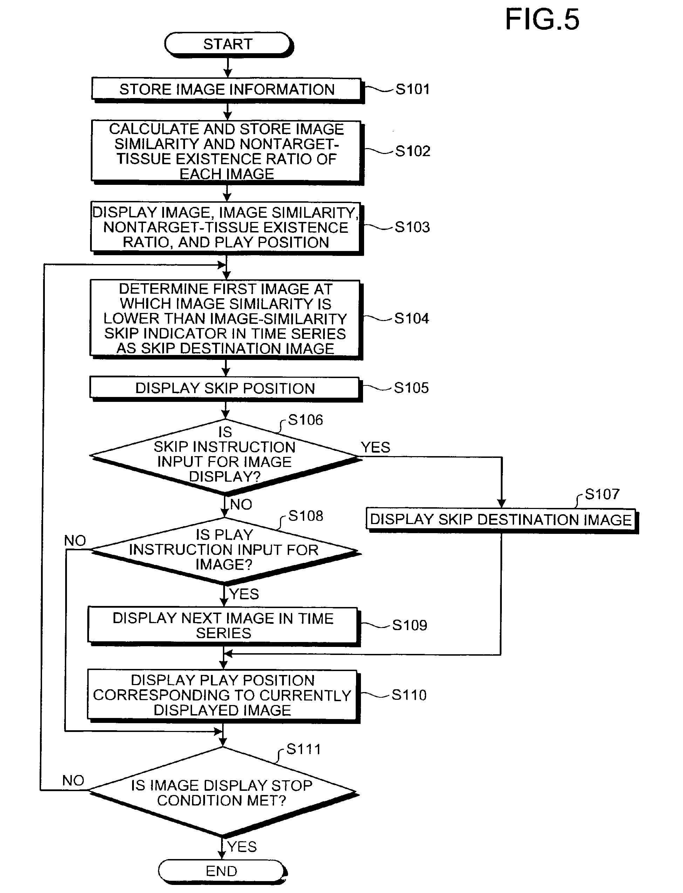

Next, a procedure to display the series of in-vivo images picked up by the capsule endoscope 2, performed by the respective units of the control unit 10 is described with reference to FIG. 5. Firstly, the control unit 10 acquires the image information of the series of in-vivo images, and stores the image information in the image information storage unit 121 (Step S101). The image-similarity calculating unit 102 calculates the image similarity of each image, and the nontarget-tissue existence-ratio calculating unit 103 calculates the nontarget-tissue existence ratio of each image, and then the information is stored in the corresponding unit of the feature-information storage unit 122 (Step S102). The display control unit 111 acquires the image information of the series of in-vivo images, the image similarity, and the nontarget-tissue existence ratio, and displays the information in the corresponding areas of the display unit 13 (Step S103). In the process described above, the image display control unit 112 displays, for example, a first image in the series of in-vivo images in the image display area F1, and the feature-information display control unit 113 displays the play position F11 corresponding to the image displayed in the image display area F1. Further, the display control unit 111 displays the image ID information together with the image and the like, on the display unit 13. At this point, the examiner can grasp how the feature information of the series of in-vivo images changes over time.

Next, the display control unit 111 determines an image whose image similarity is lower than the image-similarity skip indicator for the first time in time series since a currently displayed image to be the skip destination image (Step S104). The display control unit 111 determines the skip destination image based on the image-similarity skip indicator that is set by the examiner or automatically set by the display control unit 111. Next, the feature-information display control unit 113 displays the skip forward position F12 and the skip backward position F13 corresponding to the skip destination images (Step S105).

The display control unit 111 determines whether a skip instruction is input or not (Step S106). If the skip instruction is input (Step S106: Yes), the image display control unit 112 displays the skip destination image in the image display area F1, and proceeds to a process at Step S110 (Step S107). On the other hand, if the skip instruction is not input (Step S106: No), the display control unit 111 determines whether the play instruction for the series of in-vivo images is input or not (Step S108). If the play instruction is input (Step S108: Yes), the image display control unit 112 displays in the image display area F1, an image which is subsequent to the image currently displayed in the image display area F1 in the order of the time series (Step S109). Then, the feature-information display control unit 113 displays the play position F11 corresponding to the image displayed in the image display area F1 (Step S110). On the other hand, if the play instruction is not input (Step S108: No), the display control unit 111 does not update the image nor the play position, and proceeds to a process at Step S111.

The display control unit 111 determines whether an image display stop condition is met (Step S111). For example, the display control unit 111 determines whether an image display stop instruction is received, or the play position F11 reaches an end of a play time-series in the forward direction. While the image display stop condition is not met (Step S111: No), the display control unit 111 repeats processes at Steps S104 to S110. On the other hand, if the image display stop condition is determined to be met (Step S111: Yes), the display control unit 111 stops displaying the image.

In the process at Step S102, the image-similarity calculating unit 102 calculates the image similarity between two images picked up successively, using normalized cross-correlation described, for example, in Nonpatent Literature 1. The calculated image similarity takes a value within the range from -1 to 1. When two images are identical, the image similarity takes the maximum value, 1. A manner of calculation of the image similarity is not limited to the normalized cross-correlation, and for example, a manner described in Patent Document 2, which uses a motion vector, may be used.

In the process at Step S102, the nontarget-tissue existence-ratio calculating unit 103 divides an image area of each image into a predetermined number of pieces, for example, as described in Patent Document 3. The nontarget-tissue existence-ratio calculating unit 103 calculates, for each divided area, a plurality of feature quantities based on color tone information and texture information, specifies the living body appearing in each area using the feature quantities, and calculates the nontarget-tissue existence ratio of each image.

During the processes at Steps S103 to S111, regardless of whether an image is played or paused, the display control unit 111 is ready to receive each instruction from the forward button F3, the backward button F4, the skip forward button F5, and the skip backward button F6 through the input unit 14 as needed. Further, during the processes at steps S103 to S111, the display control unit 111 is ready to receive a change of the image-similarity skip indicator F10 as needed.

As described, in the first embodiment, the examiner sets the skip indicator as needed, knowing the image similarity and the nontarget-tissue existence ratio of a currently-displayed image and of each image. The examiner inputs the skip instruction, knowing which image would be the skip destination image and the skipped image when the skip instruction is input at the current moment. Therefore, with the image processing apparatus 5, the examiner can skip the display of images not corresponding to desired feature information, and display images corresponding to the desired feature information, confirming a correspondence between the image and the feature information. Thus, with the image processing apparatus 5, observation time for the series of in-vivo images can be shortened as images not corresponding to the desired information are skipped and not displayed. Further, as the image processing apparatus 5 reliably displays images corresponding to the desired feature information, work burden caused from rewinding or reviewing of images can be lightened.

In the first embodiment, when the skip instruction is input, the skip destination image is displayed, and the skipped image is skipped. Alternatively, the image processing apparatus may start playing images from an image that is a predetermined number of images previous to the skip destination image in the order of time series. In this case, the skip destination image is not displayed abruptly after the skip instruction, whereby an oversight of an image corresponding to desired feature information can be prevented.

Further, in the first embodiment, when the skip instruction is input, the image processing apparatus may sequentially display a series of skipped images, in other words, images starting from an image displayed at the time of reception of the skip instruction up to an image previous to the skip destination image at high speed on the image display unit 131. In this case, the examiner can confirm whether an image corresponding to the desired feature information is included in the skipped images, whereby observation time of the in-vivo images can be shortened and an oversight of an image needed for diagnosis can be prevented.

The feature information is not limited to the image similarity and the nontarget-tissue existence ratio. The feature information may be one of the image similarity and the nontarget-tissue existence ratio. When only the nontarget-tissue existence ratio is used as the feature information, a threshold value is set for the nontarget-tissue existence ratio of the skipped image as the skip indicator. In this case, an image whose nontarget-tissue existence ratio is higher than the threshold value is determined to be the skipped image. This is because, in an image whose nontarget-tissue existence ratio is high, a nontarget tissue of observation occupies a large image area and a target tissue of observation is difficult to observe. The examiner compares the nontarget-tissue existence ratio of the currently-displayed image with the nontarget-tissue existence ratio of each image, and determines to what extent a nontarget tissue should occupy an image area in the image determined to be the skipped image, in order to set the skip indicator.

Second Embodiment

Now, in the first embodiment described above, the image similarity and the nontarget-tissue existence ratio are displayed as the feature information of the series of in-vivo images, and the skipped image is determined based on the image-similarity skip indicator. In the second embodiment, the image similarity and a lesion-existence probability are displayed as the feature information. The skipped image is determined based on a lesion-existence-probability skip indicator, which is an indicator to determine the skipped image, in addition to the image-similarity skip indicator.

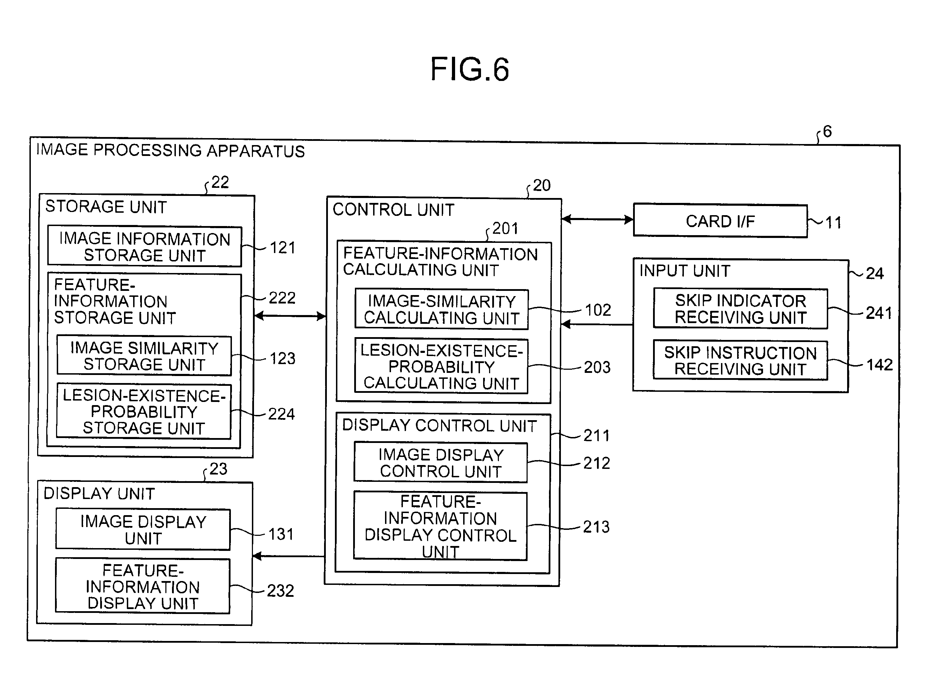

In the second embodiment, an image processing apparatus 6 (not shown) is provided in place of the image processing apparatus 5 in the intra-subject information acquiring system according to the first embodiment. FIG. 6 is a block diagram of a configuration of the image processing apparatus 6. As shown in FIG. 6, the image processing apparatus 6 includes a control unit 20, a storage unit 22, a display unit 23, and an input unit 24, in place of the control unit 10, the storage unit 12, the display unit 13, and the input unit 14 included in the image processing apparatus 5. Other configuration of the image processing apparatus 6 is the same as that of the image processing apparatus 5, and a same numeral is attached to a same element.

The control unit 20 has a feature-information calculating unit 201 and a display control unit 211. The feature-information calculating unit 201 has a lesion-existence-probability calculating unit 203 in addition to the image-similarity calculating unit 102. The lesion-existence-probability calculating unit 203 calculates a probability that a lesion area worth noting (an area to which the examiner pays particular attention), is contained in each image. Here, an area worth noting means an area which an observer such as the examiner should focus attention among observed areas (such as a mucosal membrane area and a lesion area). For example, the area worth noting includes a portion such as an abnormal or affected portion and a moving portion. The display control unit 211 controls the display of various kinds of information, and determines the skipped image based on the skip indicator. The display control unit 211 includes an image display control unit 212 and a feature-information display control unit 213. The image display control unit 212 controls the display to play the series of in-vivo images when the play instruction is input, and controls the display to skip the skipped image when the skip instruction is input. The feature-information display control unit 213 controls the display to display the image similarity and the lesion-existence probability, i.e., the feature information.

The storage unit 22 includes the image information storage unit 121 and a feature-information storage unit 222 having the image-similarity storage unit 123 and a lesion-existence-probability storage unit 224 that stores the lesion-existence probability of each image. The display unit 23 includes the image display unit 131 and a feature-information display unit 232 that displays the image similarity and the lesion-existence probability. The input unit 24 includes the skip instruction receiving unit 142 and a skip indicator receiving unit 241 that receives the image-similarity skip indicator and the lesion-existence-probability skip indicator.

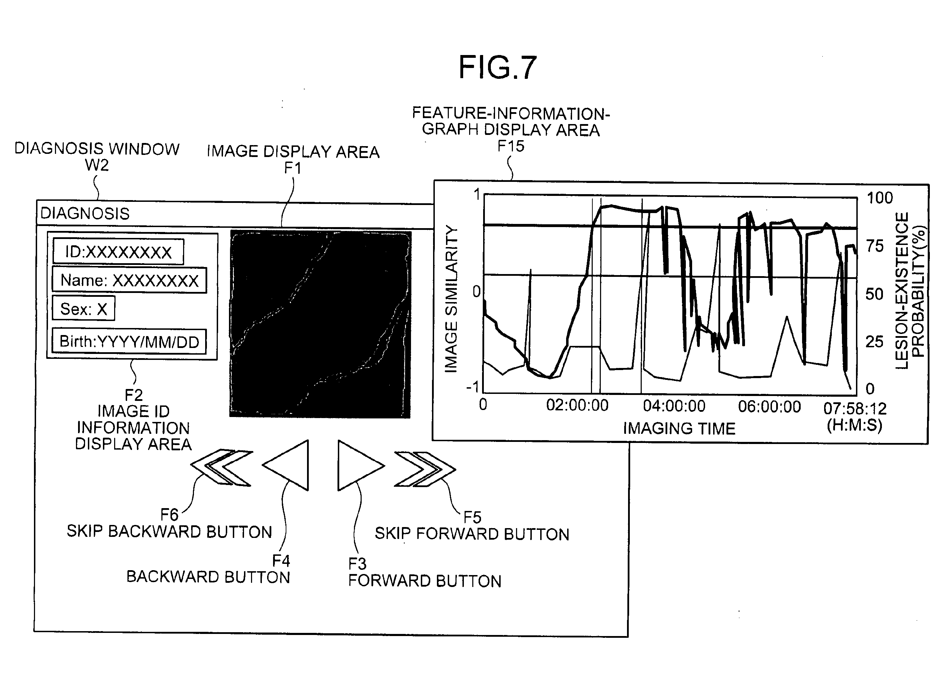

FIG. 7 is a view of an example of a display screen of the display unit 23. As shown in FIG. 7, a diagnosis window W2 displays a feature-information-graph display area F15 in place of the feature-information-graph display area F7 displayed in the diagnosis window W1. The feature-information-graph display area F15 represents a specific mode of the feature-information display unit 232. Other configurations displayed on the diagnosis window W2 are the same as the configurations of the diagnosis window W1, and a same numeral is attached to a same element.

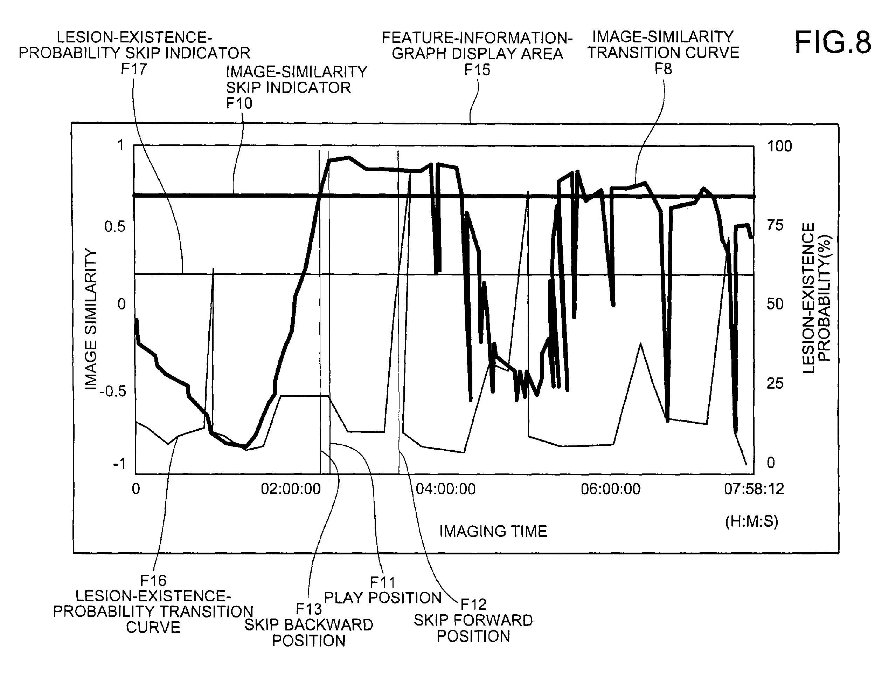

As shown in FIG. 8, in the feature-information-graph display area F15, a lesion-existence-probability transition curve F16 is displayed in place of the nontarget-tissue existence-ratio transition curve F9, and further a lesion-existence-probability skip indicator F17 is displayed. Other configurations of the feature-information-graph display area F15 are the same as the configurations of the feature-information-graph display area F7, and a same numeral is attached to a same element.

Regardless of a magnitude of the image similarity, an image with a high probability of containing a lesion area is an image the examiner desires to observe. Therefore, the examiner first checks the lesion-existence probability of each image in the series of in-vivo images referring to the lesion-existence-probability transition curve F16, and then determines to what extent the lesion-existence probability should be high for an image to be determined as the skipped image. Specifically, the examiner sets the lesion-existence-probability skip indicator as a threshold value of the lesion-existence probability to determine the skipped image.

As a specific manipulation, the examiner drags the lesion-existence-probability skip indicator F17, which is displayed as a line parallel to the horizontal axis, downward along the vertical axis using a mouse (not shown) of the input unit 24, similarly to the input of the image-similarity skip indicator. Then, the examiner drops the lesion-existence-probability skip indicator F17 at a position indicating a desired lesion-existence probability, so as to input the lesion-existence-probability skip indicator to the display control unit 211. Here, the image-similarity skip indicator F10, the lesion-existence-probability skip indicator F17, and the mouse not shown represent a specific mode of the skip indicator receiving unit 241.

The display control unit 211 determines an image whose image similarity is higher than the image-similarity skip indicator F10 and whose lesion-existence probability is lower than the lesion-existence-probability skip indicator F17, to be the skipped image. In other words, the display control unit 211 determines an image not to be skipped according to the image similarity and the lesion-existence probability, as a skip destination image.

For example, as shown in FIG. 8, an image corresponding to the skip forward position F12 is an image at which the lesion-existence probability is higher than the lesion-existence-probability skip indicator F17 for the first time in the forward direction of the imaging time-series after the play position F11, and thus is determined to be the skipped image. In this case, there is no image whose image similarity is lower than the image-similarity skip indicator F10 between the play position F11 and the skip forward position F12. On the other hand, an image corresponding to the skip backward position F13 is an image at which the image similarity is lower than the image-similarity skip indicator F10 for the first time in the backward direction of the imaging time-series from the play position F11, and thus is determined to be the skipped image. In this case, there is no image whose lesion-existence probability is higher than the lesion-existence-probability skip indicator F17 between the play position F11 and the skip backward position F13.

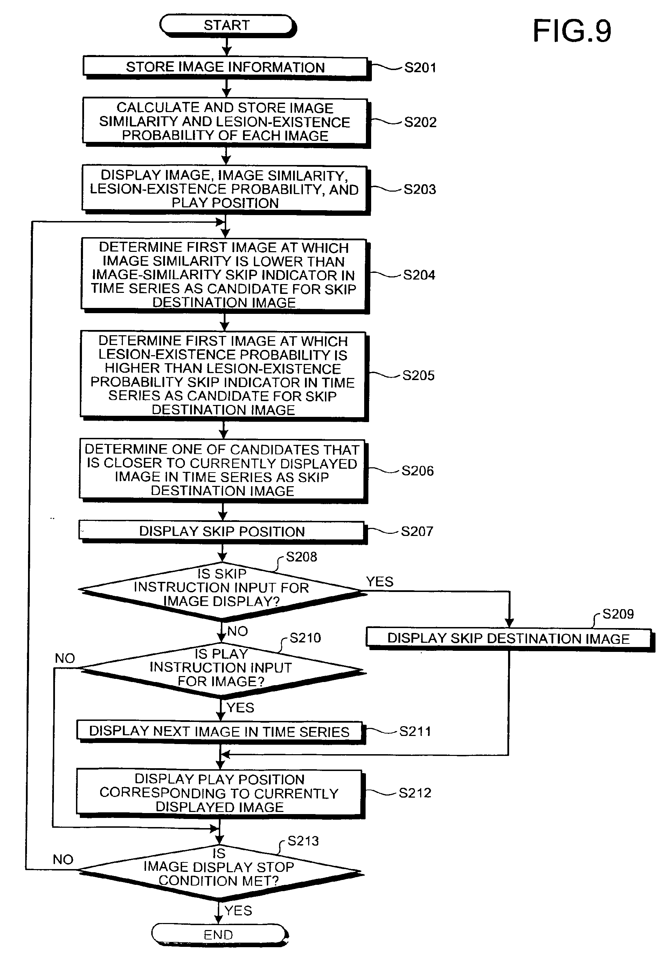

Next, a procedure to display the series of in-vivo images picked up by the capsule endoscope 2, performed by the control unit 20 is described with reference to FIG. 9. Firstly, the control unit 20 acquires the series of in-vivo images, and stores the images in the image information storage unit 121, similarly to the first embodiment (Step S201). The image-similarity calculating unit 102 calculates the image similarity of each image and the lesion-existence-probability calculating unit 203 calculates the lesion-existence probability of each image, and then the image similarity and the lesion-existence probability are stored in respective units of the feature-information storage unit 222 (Step S202). The display control unit 211 acquires the image information, the image similarity, the lesion-existence probability, and the like of the series of in-vivo images, and displays the acquired information in the corresponding areas of the display unit 23 (Step S203). Here, the image display control unit 212 displays, for example, the first picked-up image in the series of in-vivo images in the image display area F1, and the feature-information display control unit 213 displays the play position F11 corresponding to the image displayed in the image display area F1. The display control unit 211 displays the image ID information together with the image and the like on the display unit 23. At this point, the examiner can grasp how the feature information of the series of in-vivo images changes over time.

The display control unit 211 determines an image whose image similarity is lower than the image-similarity skip indicator for the first time after the currently-displayed image in the time series, to be a candidate of the skip destination image (Step S204). Further, the display control unit 211 determines an image whose lesion-existence probability is higher than the lesion-existence-probability skip indicator for the first time after the currently-displayed image in the time series, to be a candidate of the skip destination image (Step S205). Then, the display control unit 211 determines an image, which is closer to the currently-displayed image in each of the forward direction and the backward direction of the imaging time series, among the skip destination image candidates, to be the skip destination image (Step S206). The feature-information display control unit 213 displays the skip forward position F12 and the skip backward position F13 corresponding to the skip destination images (step S207).

Procedures to display the series of in-vivo images (Steps S208 to S213) are the same as procedures at steps S106 to S111 in the first embodiment. If the skip instruction is input (Step S208: Yes), the image display control unit 212 displays the skip destination image in the image display area F1 (Step S209). If the skip instruction is not input (Step S208: No) and the play instruction is input (Step S10: Yes), the image display control unit 212 displays in the image display area F1, an image that is picked up after the image currently displayed in the image display area F1 in the time series (Step S211). Next, the feature-information display control unit 213 displays the play position F11 corresponding to the image displayed in the image display area F1 (step S212). After the step S212 or when the play instruction is not input at Step S210 (Step S210: No), the display control unit 211 repeats the processes at Steps S204 to S212 until the image display stop condition is met (Step S213). During the processes at Steps S203 to S213, the display control unit 211 is ready to receive the play instruction, the skip instruction, and the skip indicator change as needed, similarly to the display control unit 111.

At Step S202, the lesion-existence-probability calculating unit 203 calculates the lesion-existence probability of each image through processes described below. Firstly, the lesion-existence-probability calculating unit 203 acquires feature quantity of an image containing a lesion and a normal organ tissue inside a body before performing the process at Step S202. To be specific, the lesion-existence-probability calculating unit 203 acquires a feature quantity of a lesion area extracted from plural sample images for each kind of lesions such as bleeding and mucosal discoloration, as training data of the lesion area. The lesion-existence-probability calculating unit 203 also acquires beforehand a feature quantity of a normal area extracted from plural sample images, as training data of a normal organ tissue area inside the body (referred to below as "normal area"). Here, the feature quantity is R, G, and B values (R, G, and B values are together referred to below as "pixel value") of each pixel within each image area. Further, the lesion-existence-probability calculating unit 203 divides the lesion areas into groups according to the type of lesion, and divides the normal areas by grouping those with a similar distribution of pixel values into the same group. Then, the lesion-existence-probability calculating unit 203 acquires an average pixel value and a pixel value covariance of each group.

After that, as the process at Step S202, the lesion-existence-probability calculating unit 203 employs the distribution of feature quantities of each group in the training data as a normal distribution, and calculates a probability that each pixel of each image in the series of in-vivo images belongs to each group, using the average pixel value and the pixel value covariance of each group, and following formula (1).

[Formula 1]

$$p(k \mid x) = \frac{1}{(2\pi)^{n/2}|\Sigma_k|^{1/2}} \exp\left\{-\frac{1}{2}(x - \mu_k)^t \Sigma^{-1}(x - \mu_k)\right\} \qquad (1)$$

In the formula (1), k represents group number, $\mu_k$ represents average pixel value, $\Sigma_k$ represents pixel value covariance, x represents pixel value of each pixel, n represents number of dimensions, and t represents transposition. When x = (RVal, GVal, BVal), n = 3.

The lesion-existence-probability calculating unit 203 determines that each pixel belongs to a group for which the probability p(k|x) that the pixel belongs to the group is highest. The lesion-existence-probability calculating unit 203 performs a labeling process, which is described in Nonpatent Literature 2, on a pixel determined to belong to the lesion group, for each image. The lesion-existence-probability calculating unit 203 creates lesion area(s) by classifying the pixels belonging to the same lesion group into a same lesion area. The lesion-existence-probability calculating unit 203 calculates, for each lesion area of each image, an average probability that each pixel belongs to the lesion group (referred to below as "lesion area probability"). After that, the lesion-existence-probability calculating unit 203 determines a highest lesion area probability among all lesion area probabilities of each image as the lesion-existence probability of the image. The feature quantity is not limited to the R, G, and B values, and the feature quantity may be a color ratio, a shape feature quantity, or a combination thereof.

As described above, in the second embodiment, the examiner can grasp the lesion-existence probability as well as the image similarity, and set the skip indicator of the image similarity and the lesion-existence probability. Therefore, with the image processing apparatus 6, the examiner can determine the skipped image based on both the image similarity and the lesion-existence probability, whereby images other than desired images can be skipped and the desired images can be displayed more reliably.

In the second embodiment, similarly to the first embodiment, when the skip instruction is input, the image processing apparatus may start playing images from an image that is a predetermined number of images previous to the skip destination image in the play time-series. Alternatively, the image processing apparatus may display the skipped image at high speed before displaying the skip destination image.

In the second embodiment, the skip indicator is set for the image similarity and the lesion-existence probability displayed, as the feature information of the image. Alternatively, the image processing apparatus may display one of the image similarity and the lesion-existence probability, and set only the skip indicator corresponding to the displayed feature information in order to determine the skipped image.

In the first and second embodiments, the probability that the lesion area which is the area worth noting appears in the image is displayed. Alternatively, however, a probability that the area worth noting, which is an area the examiner focuses, such as a mucosal membrane area, appears in the image may be displayed.

INDUSTRIAL APPLICABILITY

As described above, the image processing apparatus, image processing program, and image processing method according to the invention are useful for displaying in-vivo images in which an interior of a living body is captured.

**Claims**

1. An image processing apparatus comprising:

an image storage unit that stores an image;
an image display unit that sequentially displays each image;
a feature-information storage unit that stores feature information of each image;
a feature-information display unit that displays the feature information;
a skip indicator receiving unit that receives a skip indicator, which is an indicator set in relation to the feature information to skip a display of the image on the image display unit;
a skip instruction receiving unit that receives an instruction to skip the image displayed on the image display unit; and
an image display control unit that controls the image display unit to skip the image displayed on the image display unit according to the feature information and based on the skip indicator when the skip instruction receiving unit receives the instruction to skip an image.

2. The image processing apparatus according to claim 1, wherein
the skip indicator receiving unit receives a threshold value of the feature information as the skip indicator, and
the feature-information display unit displays the skip indicator.

3. The image processing apparatus according to claim 1 or 2, wherein
the feature-information display unit displays an indicator indicating a position of a skip destination image determined according to the skip indicator, in time series of all the images.

4. The image processing apparatus according to any one of claims 1 to 3, wherein
the image display control unit controls the image display unit to sequentially display the images starting from an image, which is a predetermined number of images previous to the skip destination image in the time series, when receiving the instruction to skip the display of an image.

5. The image processing apparatus according to any one of claims 1 to 4, wherein
the image display control unit controls, when receiving the instruction to skip the display of an image, the image display unit to sequentially display a series of images at high speed starting from an image displayed on the image display unit when the instruction is received up to the skip destination image.

6. The image processing apparatus according to any one of claims 1 to 5, wherein
the feature-information display unit displays a degree of similarity of images substantially sequential in time series among the images, a probability that an area worth noting appears in each of the images, and a ratio of an area occupied by an object, which is not a target of observation, in each of the images, or a combination thereof as the feature information.

7. The image processing apparatus according to any one of claims 1 to 6, wherein
the feature-information display unit displays the feature information in association with play time that indicates time required for sequentially displaying the series of images, imaging time that indicates time when each image is picked up, an image number that is attached to each image in an order of imaging, or a combination thereof.

8. The image processing apparatus according to any one of claims 1 to 7, wherein
the feature-information display unit displays an indicator indicating a position of an image currently displayed on the image display unit in a time series of the series of images.

9. The image processing apparatus according to claim 6, wherein
the area worth noting is a lesion area.

10. The image processing apparatus according to any one claims 1 to 9, wherein
the image is an in-vivo image in which an image of an interior of a living body is captured.

11. An image processing program for reading each of images stored in an image storage unit and sequentially displaying the images on an image display unit, causing a computer to perform:

   a feature information storing step of storing feature information of each of the images stored in the image storage unit, in a feature-information storage unit;
   a feature information displaying step of displaying the feature information;
   a skip indicator receiving step of receiving a skip indicator, which is an indicator set in relation to the feature information to skip a display of the image on the image display unit;
   a skip instruction receiving step of receiving an instruction to skip the image displayed on the image display unit; and
   an image display controlling step of controlling the image display unit to skip the image displayed on the image display unit according to the feature information and based on the skip indicator when the instruction to skip the image is received in the skip instruction receiving step.

12. An image processing method for reading each of images stored in an image storage unit and sequentially displaying the images on an image display unit, comprising:

a feature information storing step of storing feature information of each of the images stored in the image storage unit, in a feature-information storage unit;

a feature information displaying step of displaying the feature information;

a skip indicator receiving step of receiving a skip indicator, which is an indicator set in relation to the feature information to skip a display of the image on the image display unit;

a skip instruction receiving step of receiving an instruction to skip the image displayed on the image display unit; and

an image display controlling step of controlling the image display unit to skip the image displayed on the image display unit according to the feature information and based on the skip indicator when the instruction to skip the image is received in the skip instruction receiving step.

# FIG.1

CAPSULE ENDOSCOPE
2

1
SUBJECT

IMAGE PROCESSING
APPARATUS
5

3
RECEIVING
APPARATUS

4
RECORDING MEDIUM

# FIG.2

IMAGE PROCESSING APPARATUS — 5

STORAGE UNIT — 12

IMAGE INFORMATION STORAGE UNIT — 121

FEATURE-INFORMATION STORAGE UNIT — 122

IMAGE SIMILARITY STORAGE UNIT — 123

NONTARGET-TISSUE EXISTENCE-RATIO STORAGE UNIT — 124

CONTROL UNIT — 10

FEATURE-INFORMATION CALCULATING UNIT — 101

IMAGE-SIMILARITY CALCULATING UNIT — 102

NONTARGET-TISSUE EXISTENCE-RATIO CALCULATING UNIT — 103

DISPLAY CONTROL UNIT — 111

IMAGE DISPLAY CONTROL UNIT — 112

FEATURE-INFORMATION DISPLAY CONTROL UNIT — 113

CARD I/F — 11

INPUT UNIT — 14

SKIP INDICATOR RECEIVING UNIT — 141

SKIP INSTRUCTION RECEIVING UNIT — 142

DISPLAY UNIT — 13

IMAGE DISPLAY UNIT — 131

FEATURE-INFORMATION DISPLAY UNIT — 132

EP 2 181 642 A1

# FIG.3

FEATURE-INFORMATION-GRAPH DISPLAY AREA
F7

DIAGNOSIS WINDOW
W1

IMAGE DISPLAY AREA
F1

DIAGNOSIS

ID:XXXXXXXX

Name:XXXXXXXX

Sex:X

Birth:YYYY/MM/DD

F2
IMAGE ID
INFORMATION
DISPLAY AREA

F6
SKIP BACKWARD
BUTTON

F4
BACKWARD BUTTON

F3
FORWARD BUTTON

F5
SKIP FORWARD
BUTTON

IMAGE SIMILARITY

NONTARGET-TISSUE EXISTENCE RATIO(%)

IMAGING TIME

07:58:12
(H:M:S)

EP 2 181 642 A1

FIG.4

IMAGE-SIMILARITY SKIP INDICATOR F10

IMAGE-SIMILARITY SKIP INDICATOR (BEFORE CHANGE) F14

FEATURE-INFORMATION-GRAPH DISPLAY AREA F7

IMAGE-SIMILARITY TRANSITION CURVE F8

F9 NONTARGET-TISSUE EXISTENCE-RATIO TRANSITION CURVE

F13 SKIP BACKWARD POSITION

F11 PLAY POSITION

F12 SKIP FORWARD POSITION

IMAGE SIMILARITY

NONTARGET-TISSUE EXISTENCE RATIO(%)

IMAGING TIME

(H:M:S)

EP 2 181 642 A1

# FIG.5

```
                    ( START )
                        │
                        ▼
        ┌──────────────────────────────┐
        │   STORE IMAGE INFORMATION     │────S101
        └──────────────────────────────┘
                        │
                        ▼
        ┌──────────────────────────────┐
        │  CALCULATE AND STORE IMAGE    │
        │  SIMILARITY AND NONTARGET-    │────S102
        │  TISSUE EXISTENCE RATIO OF    │
        │         EACH IMAGE            │
        └──────────────────────────────┘
                        │
                        ▼
        ┌──────────────────────────────┐
        │ DISPLAY IMAGE, IMAGE SIMILARITY, │
        │ NONTARGET-TISSUE EXISTENCE    │────S103
        │ RATIO, AND PLAY POSITION      │
        └──────────────────────────────┘
                        │
                        ▼
        ┌──────────────────────────────┐
        │  DETERMINE FIRST IMAGE AT     │
        │  WHICH IMAGE SIMILARITY IS    │
        │  LOWER THAN IMAGE-SIMILARITY  │────S104
        │  SKIP INDICATOR IN TIME SERIES│
        │  AS SKIP DESTINATION IMAGE    │
        └──────────────────────────────┘
                        │
                        ▼
        ┌──────────────────────────────┐
        │    DISPLAY SKIP POSITION      │────S105
        └──────────────────────────────┘
                        │
                        ▼
                  ◇ S106
              IS SKIP INSTRUCTION          YES
              INPUT FOR IMAGE        ─────────────►  ┌────────────────────────────────┐
                 DISPLAY?                            │ DISPLAY SKIP DESTINATION IMAGE │──S107
                    │ NO                             └────────────────────────────────┘
                    ▼
                  ◇ S108
        NO     IS PLAY
        ◄───── INSTRUCTION INPUT FOR
               IMAGE?
                    │ YES
                    ▼
        ┌──────────────────────────────┐
        │  DISPLAY NEXT IMAGE IN TIME   │────S109
        │           SERIES              │
        └──────────────────────────────┘
                        │
                        ▼
        ┌──────────────────────────────┐
        │  DISPLAY PLAY POSITION        │
        │  CORRESPONDING TO CURRENTLY   │────S110
        │  DISPLAYED IMAGE              │
        └──────────────────────────────┘
                        │
                        ▼
                  ◇ S111
        NO     IS IMAGE DISPLAY STOP
        ◄───── CONDITION MET?
                    │ YES
                    ▼
                  ( END )
```

# FIG.6

IMAGE PROCESSING APPARATUS  6

STORAGE UNIT  22

IMAGE INFORMATION STORAGE UNIT  121

FEATURE-INFORMATION STORAGE UNIT  222

IMAGE SIMILARITY STORAGE UNIT  123

LESION-EXISTENCE-PROBABILITY STORAGE UNIT  224

CONTROL UNIT  20

FEATURE-INFORMATION CALCULATING UNIT  201

IMAGE-SIMILARITY CALCULATING UNIT  102

LESION-EXISTENCE-PROBABILITY CALCULATING UNIT  203

DISPLAY CONTROL UNIT  211

IMAGE DISPLAY CONTROL UNIT  212

FEATURE-INFORMATION DISPLAY CONTROL UNIT  213

CARD I/F  11

INPUT UNIT  24

SKIP INDICATOR RECEIVING UNIT  241

SKIP INSTRUCTION RECEIVING UNIT  142

DISPLAY UNIT  23

IMAGE DISPLAY UNIT  131

FEATURE-INFORMATION DISPLAY UNIT  232

EP 2 181 642 A1

19

# FIG.7

EP 2 181 642 A1

FEATURE-INFORMATION-
GRAPH DISPLAY AREA
F15

DIAGNOSIS WINDOW
W2

IMAGE DISPLAY AREA
F1

DIAGNOSIS

ID:XXXXXXXX

Name: XXXXXXXX

Sex: X

Birth:YYYY/MM/DD

F2
IMAGE ID
INFORMATION
DISPLAY AREA

F6
SKIP BACKWARD BUTTON

F4
BACKWARD BUTTON

F3
FORWARD BUTTON

F5
SKIP FORWARD BUTTON

IMAGE SIMILARITY

LESION-EXISTENCE
PROBABILITY(%)

1

0

-1

100

75

50

25

0

0    02:00:00    04:00:00    06:00:00    07:58:12
IMAGING TIME                              (H:M:S)

FIG.8

LESION-EXISTENCE-
PROBABILITY SKIP INDICATOR
F17

IMAGE-SIMILARITY
SKIP INDICATOR
F10

FEATURE-INFORMATION-
GRAPH DISPLAY AREA
F15

IMAGE-SIMILARITY
TRANSITION CURVE
F8

IMAGE SIMILARITY

LESION-EXISTENCE
PROBABILITY(%)

IMAGING TIME

(H:M:S)

F16
LESION-EXISTENCE-
PROBABILITY TRANSITION
CURVE

F13
SKIP BACKWARD
POSITION

F11
PLAY POSITION

F12
SKIP FORWARD
POSITION

EP 2 181 642 A1

FIG.9

```
                    ┌─────────────┐
                    │    START    │
                    └──────┬──────┘
                           ▼
          ┌────────────────────────────────┐
          │   STORE IMAGE INFORMATION       │──S201
          └────────────────┬───────────────┘
                           ▼
          ┌────────────────────────────────┐
          │ CALCULATE AND STORE IMAGE       │
          │ SIMILARITY AND LESION-EXISTENCE │──S202
          │ PROBABILITY OF EACH IMAGE       │
          └────────────────┬───────────────┘
                           ▼
          ┌────────────────────────────────┐
          │ DISPLAY IMAGE, IMAGE SIMILARITY,│
          │ LESION-EXISTENCE PROBABILITY, AND│──S203
          │ PLAY POSITION                   │
          └────────────────┬───────────────┘
                           ▼
          ┌────────────────────────────────┐
          │ DETERMINE FIRST IMAGE AT WHICH  │
          │ IMAGE SIMILARITY IS LOWER THAN  │
          │ IMAGE-SIMILARITY SKIP INDICATOR IN│──S204
          │ TIME SERIES AS CANDIDATE FOR SKIP│
          │ DESTINATION IMAGE               │
          └────────────────┬───────────────┘
                           ▼
          ┌────────────────────────────────┐
          │ DETERMINE FIRST IMAGE AT WHICH  │
          │ LESION-EXISTENCE PROBABILITY IS │
          │ HIGHER THAN LESION-EXISTENCE    │
          │ PROBABILITY SKIP INDICATOR IN TIME│──S205
          │ SERIES AS CANDIDATE FOR SKIP    │
          │ DESTINATION IMAGE               │
          └────────────────┬───────────────┘
                           ▼
          ┌────────────────────────────────┐
          │ DETERMINE ONE OF CANDIDATES THAT│
          │ IS CLOSER TO CURRENTLY DISPLAYED│──S206
          │ IMAGE IN TIME SERIES AS SKIP    │
          │ DESTINATION IMAGE               │
          └────────────────┬───────────────┘
                           ▼
          ┌────────────────────────────────┐
          │    DISPLAY SKIP POSITION        │──S207
          └────────────────┬───────────────┘
                           ▼
                    ╱─────────────╲  S208
                   ╱  IS SKIP      ╲      YES
                  ╱ INSTRUCTION INPUT ╲──────────┐
                  ╲ FOR IMAGE DISPLAY?╱          │
                   ╲───────┬─────────╱           ▼
                        NO │              ┌──────────────────────────┐
                           ▼              │ DISPLAY SKIP DESTINATION │──S209
                    ╱─────────────╲  S210 │ IMAGE                    │
                   ╱  IS PLAY      ╲      └────────────┬─────────────┘
              NO  ╱ INSTRUCTION INPUT╲                 │
           ┌────╱    FOR IMAGE?      ╲                 │
           │    ╲───────┬─────────────╱                │
           │         YES│                              │
           │            ▼                              │
           │  ┌──────────────────────────────┐         │
           │  │ DISPLAY NEXT IMAGE IN TIME SERIES│──S211 │
           │  └────────────┬─────────────────┘         │
           │               ◄──────────────────────────┘
           │               ▼
           │  ┌──────────────────────────────┐
           │  │ DISPLAY PLAY POSITION         │
           │  │ CORRESPONDING TO CURRENTLY    │──S212
           │  │ DISPLAYED IMAGE               │
           │  └────────────┬─────────────────┘
           │               ▼
           │        ╱─────────────╲  S213
           │  NO   ╱      IS       ╲
           └──────╱ IMAGE DISPLAY STOP╲
                  ╲  CONDITION MET?   ╱
                   ╲───────┬─────────╱
                        YES│
                           ▼
                    ┌─────────────┐
                    │     END     │
                    └─────────────┘
```

## INTERNATIONAL SEARCH REPORT

| | International application No. |
|---|---|
| | PCT/JP2008/060301 |

**A. CLASSIFICATION OF SUBJECT MATTER**
*A61B1/04*(2006.01)i, *A61B1/00*(2006.01)i

According to International Patent Classification (IPC) or to both national classification and IPC

**B. FIELDS SEARCHED**

Minimum documentation searched (classification system followed by classification symbols)
A61B1/04, A61B1/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2006-149684 A (Olympus Corp.), 15 June, 2006 (15.06.06), Par. Nos. [0065], [0090], [0092]; Fig. 9 & US 2008/0024599 A & WO 2006/057193 A1 & CN 101065050 A | 1,3,5-12 |
| Y | WO 2006/019120 A1 (Olympus Corp.), 23 February, 2006 (23.02.06), Par. Nos. [0038], [0039], [0047], [0059]; Fig. 5 & EP 1787574 A1 & WO 2006/019120 A1 & CN 101005795 A | 1,3,4,6-12 |

| ☒ Further documents are listed in the continuation of Box C. | ☐ See patent family annex. |
|---|---|

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| 11 August, 2008 (11.08.08) | 19 August, 2008 (19.08.08) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.

PCT/JP2008/060301

C (Continuation).    DOCUMENTS CONSIDERED TO BE RELEVANT

| Category* | Citation of document, with indication, where appropriate, of the relevant passages | Relevant to claim No. |
|---|---|---|
| Y | JP 2006-296569 A  (Olympus Medical Systems Corp.),<br>02 November, 2006 (02.11.06),<br>Par. Nos. [0038], [0039]; Fig. 4<br>(Family: none) | 1-3,6-12 |
| Y | JP 2006-334297 A  (Olympus Medical Systems Corp.),<br>14 December, 2006 (14.12.06),<br>Par. No. [0045]; Fig. 5<br>(Family: none) | 1-12 |
| Y | JP 2005-211535 A  (Olympus Corp.),<br>11 August, 2005 (11.08.05),<br>Par. No. [0028]<br>& US 2005/0261550 A1 | 1,2,4-6,9-12 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2006330376 A
- JP 2006280792 A
- JP 2006288879 A

### Non-patent literature cited in the description

- Digital Image Processing. CG-ARTS Society, 22 July 2004, 202
- Digital Image Processing. CG-ARTS Society, 22 July 2004, 181